(19) Europäisches Patentamt
European Patent Office
Office européen des brevets

(11) Veröffentlichungsnummer: **0 288 028**
**A2**

# (12) EUROPÄISCHE PATENTANMELDUNG

(21) Anmeldenummer: **88106296.2**

(22) Anmeldetag: **20.04.88**

(51) Int. Cl.⁴: **C07D 207/267 , C07C 143/78 , A61K 31/40 , A61K 31/18**

Patentansprüche für folgende Vertragsstaaten: ES + GR.

(30) Priorität: **24.04.87 DE 3713757**

(43) Veröffentlichungstag der Anmeldung: **26.10.88 Patentblatt 88/43**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lang, Hans-Jochen, Dr.**
**Rüdesheimer Strasse 7**
**D-6238 Hofheim am Taunus(DE)**
Erfinder: **Hropot, Max, Dr.**
**Friedrich-Stolz-Strasse 13**
**D-6093 Flörsheim am Main(DE)**
Erfinder: **Granzer, Ernold, Dr. Dr.**
**Falkensteiner Strasse 24**
**D-6233 Kelkheim (Taunus)(DE)**
Erfinder: **Kerekjarto, Bela, Dr.**
**Weilbächer Wälder FA8**
**D-6238 Hofheim am Taunus(DE)**

## (54) Benzolsulfonamidderivate und Verfahren zu ihrer Herstellung.

(57) Beschrieben werden Verbindungen der Formel I

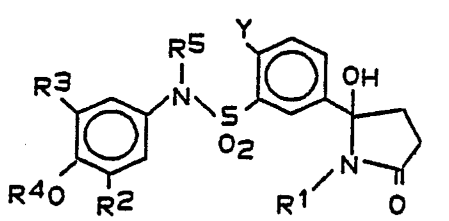

I,

mit $R^1$ gleich H, (Cyclo)Alk(en)yl, $R^2$ gleich H, Alkyl, $R^3$ gleich Alkyl, $R^4/R^5$ gleich H, Alkyl oder Acyl und Y gleich H, Alkyl, $CF_3$, Hal; ebenso werden Verfahren zu ihrer Herstellung beschrieben.
Sie sind wirkungsvolle Diuretika und Antihypertensiva mit lipidsenkender Wirkung.

EP 0 288 028 A2

## Benzolsulfonamidderivate und Verfahren zu ihrer Herstellung

Die Erfindung betrifft Benzolsulfonamidderivate.

Es ist bekannt, daß Benzolsulfonamidderivate der Formel XII

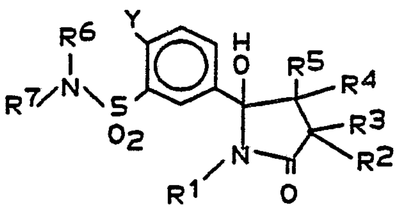

XII

eine starke diuretische und saluretische Wirkung besitzen (US-Pat. 4.235.918). In diesen bekannten Verbindungen ist jedoch $R^7$ nicht gegebenenfalls substituiertes Phenyl.

Für viele Zwecke sind diese bekannten Verbindungen noch nicht voll befriedigend, vor allem für die Behandlung der Hypertonie des älteren Menschen.

Die Erfindung hat sich deshalb die Aufgabe gestellt, Benzolsulfonamidderivate mit weiter verbesserten Eigenschaften zur Verfügung zu stellen, die mit ihrer Wirksamkeit die bekannten Verbindungen XII übertreffen.

Dies ist gelungen durch Benzolsulfonamidderivate der Formel I

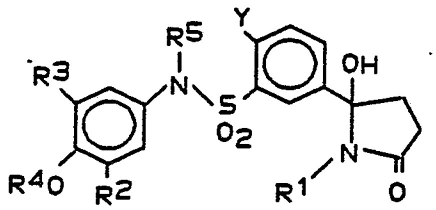

I,

worin
$R^1$ Wasserstoff, Alkyl oder Alkenyl mit 1-4 C-Atomen, Cycloalkyl mit 3 bis 5 Ringgliedern,
$R^2$ Wasserstoff oder einen Alkylrest mit 1 bis 6 C-Atomen,
$R^3$ einen Alkylrest mit 3 bis 5 C-Atomen,
$R^4$ und $R^5$ Wasserstoff, einen $(C_1$-$C_3)$-Alkylrest oder einen Acylrest einer aliphatischen Carbonsäure mit 1 bis 3 C-Atomen und
Y Wasserstoff, Methyl, Trifluormethyl, F oder Cl bedeuten, sowie die entsprechend zu I offenkettigen tautomeren Formen der Formel Ia

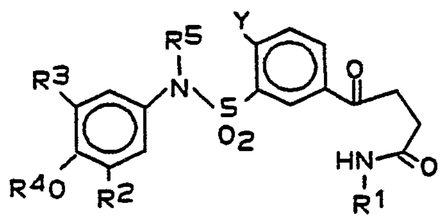

Ia

worin $R^1$ bis $R^5$ und Y die angegebene Bedeutung besitzen.

Bevorzugt sind solche Verbindungen der Formel I, worin $R^4$ und $R^5$ Wasserstoff und Y Chlor bedeuten.

Besonders bevorzugt sind Verbindungen der Formel I, worin $R^4$ und $R^5$ für Wasserstoff, Y für Chlor stehen und $R^2$ und $R^3$ Methyl, Isopropyl und/oder Tert.-Butyl bedeuten, wobei jedoch $R^3$ mindestens 3 Kohlenstoffatome enthält und verzweigt ist.

Insbesondere sind diejenigen von herausragender Bedeutung, bei denen $R^1$ in Bedeutung von Methyl steht.

Als herausragende Einzelverbindungen sind zu nennen:

5-[4-Chlor-3-(3,5-diisopropyl-4-hydroxyphenylsulfamoyl)phenyl]-5-hydroxy-1-methyl-2-pyrrolidon
5-[4-Chlor-3-(3,5-di-tert.-butyl-4-hydroxyphenylsulfamoyl)phenyl]-5-hydroxy-1-methyl-2-pyrrolidon.

Die Erfindung betrifft weiterhin ein Verfahren zur Herstellung der Verbindungen der Formel I, das dadurch gekennzeichnet ist, daß man

a) Verbindungen der Formel II

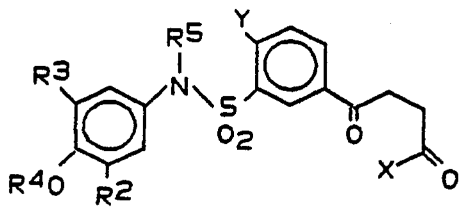

II

mit einem primären Amin der Formel III in üblicher Weise umsetzt

$R^1$-$NH_2$ III

wobei $R^1$ bis $R^5$ und Y die angegebene Bedeutung besitzen und X der reaktive anorganische oder organische Rest der Carbonsäure (X = OH) ist, oder

b) Verbindungen der Formel IV

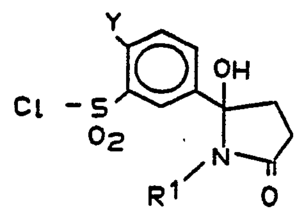

IV

mit einem Amin der Formel V

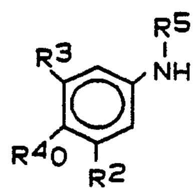

V

umsetzt, worin $R^1$ bis $R^5$ und Y die angegebene Bedeutung besitzen, und gegebenenfalls die nach Weg a) und b) erhaltenen Verbindungen der Formel I, worin $R^4$ und/oder $R^5$ Wasserstoff bedeutet, durch übliche Alkylierung oder Acylierung in Verbindungen der Formel I überführt, in denen $R^4$ und/oder $R^5$ eine der weiteren oben angegebenen Bedeutungen hat.

Die Verbindungen der Formel IV können auch analog zum Gleichgewicht I ⇄ Ia in ihren offenkettigen tautomeren Formen IVa vorliegen:

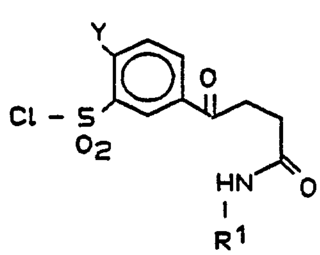

IVa

Welche der beiden tautomeren Formen I oder Ia einer erfindungsgemäßen Verbindung mit überwiegendem Anteil im nachfolgend formulierten Gleichgewicht

I ⇄ 1a

vorliegt, hängt vom Lösungsmittel, der Temperatur, insbesondere aber vom Substituenten $R^1$ ab.

Die erfindungsgemäßen Verbindungen der Formel I können außerdem in ihren möglichen geometrisch isomeren Strukturen vorliegen.

Die Alkylreste in den Substituenten $R^1$ bis $R^5$ können sowohl geradkettig als auch verzweigt sein.

Die unter a) bezeichnete Verfahrensweise wird vorteilhaft so ausgeführt, daß man beispielsweise Verbindungen der Formel II, worin $R^2$ bis $R^5$ und Y die angegebene Bedeutung besitzen, und X der O-($C_1$-$C_4$)-Acylrest eines gemischten Anhydrides ist, mit Aminen der Formel III zur Reaktion bringt. Die gemischten Anhydride werden vorteilhaft dadurch in situ erzeugt, daß man Verbindungen der Formel VI

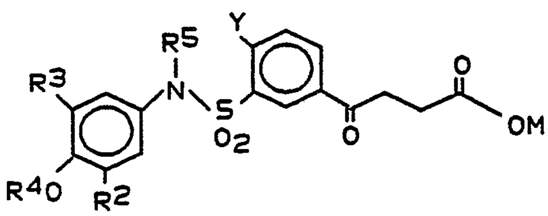

VI ,

worin M Wasserstoff bedeutet, mit einem Äquivalent einer geeigneten Base, beispielsweise einem Alkali- oder Erdalkalihydroxid wie KOH, NaOH, $Ca(OH)_2$, einem Alkalimetallcarbonat oder -hydrogencarbonat wie $Na_2CO_3$, $K_2CO_3$, $NaHCO_3$, $KHCO_3$ oder mit einem vorzugsweise tertiären Amin wie Trimethylamin, Triethylamin, Tripropylamin, Tributylamin, Dicyclohexylethylamin, in die entsprechenden Carbonsäuresalze der Formel VI überführt, worin M vorzugsweise für Na, K, Ca oder eine Trialkylammoniumkation, wie $[HN(C_2H_5)_3]^+$ steht, die anschließend mit 1-2,5 Mol, bevorzugt mit 1 bis 1,5 Mol, eines aktivierten Säurederivates zu einem gemischten Anhydrid umgesetzt werden.

Man verwendet als aktiviertes Säurederivat vorteilhaft beispielsweise einen Chlorameisensäurealkylester, bevorzugt Chlorameisensäureethylester oder Chlorameisensäuremethylester, ein Carbamidsäurechlorid (wie N,N-Dimethylcarbamidsäurechlorid oder N,N-Dimethylcarbamidsäurechlorid) oder das Säurechlorid einer aliphatischen oder aromatischen Sulfonsäure, beispielsweise Methan-, Ethan-, Benzol-oder p-Toluolsulfonsäurechlorid.

Die Reaktion führt man vorteilhaft in einem wasserfreien, polaren, organischen Lösungsmittel, beispielsweise in Aceton, Methylethylketon, einem Alkansäure-($C_1$-$C_6$)alkylester wie Essigsäuremethyl-oder -ethylester, einem Alkansäureamid, beispielsweise Dimethylformamid, Dimethylacetamid, in Dimethylsulfoxid, in Acetonitril, in einem niederen aliphatischen Alkohol, z.B. in Methanol, Ethanol oder Isopropanol, bevorzugt in einem ringförmigen oder offenkettigen Ether und Polyether, wie Tetrahydrofuran, Dioxan, Ethylenglycoldimethylether, Diethylenglycoldimethylether durch. Die Reaktionsdauer für einen 0,1 molaren Ansatz beträgt 1 Minute bis 5 Stunden, wobei man das Gemisch bevorzugt über einen Zeitraum von 5 bis 60 Minuten rühren läßt. Die Reaktion kann in einem Temperaturbereich von -50 °C bis 100 °C durchgeführt werden, wobei man vorteilhaft zwischen -30 °C und +30 °C, bevorzugt zwischen -10 °C und +15 °C arbeitet.

Obgleich die gemischten Anhydride gefaßt werden können, indem man beispielsweise das Lösungsmittel bei Temperaturen zwischen -5 °C und +10 °C verdampft und mit einem geeigneten Solvens, wie Essigsäureethylester aus dem Rückstand extrahiert, verfährt man vorteilhaft so, daß man auf die Isolierung des gemischten Anhydrides verzichtet und das Amin der Formel III zum Reaktionsgemisch gibt. Das Amin kann sowohl unverdünnt als auch in Form einer Lösung zugegeben werden, wobei man als Lösungsmittel vorteilhaft dasjenige des Reaktionsgemisches oder eines der hierfür angegebenen Solvenzien sowie auch wäßrige Lösungen des Amins oder von Ammoniak verwenden kann. Dabei kommt, bezogen auf die Verbindung der Formel VI, mindestens 1 Mol, vielfach vorteilhaft aber ein deutlicher molarer Überschuß an Amin (10-fach und mehr) zur Anwendung. Die Reaktion wird in einem Temperaturbereich zwischen -30 °C und +100 °C, bevorzugt zwischen +5 °C und +40 °C durchgeführt. Die Reaktionszeit liegt zwischen 10 Minuten und 5 Tagen, wobei der Reaktionsfortgang dünnschichtchromatografisch, vorteilhaft an Kieselgel, verfolgt werden kann.

Die aus Verbindungen der Formel VI mit $COCl_2$, Oxalylchlorid, $POCl_3$, $SOCl_2$, $PCl_3$ oder $PCl_5$ darstellbaren Säurechloride der Formel II, worin X für Chlor steht, werden prinzipiell wie die gemischten Anhydride in der eben beschriebenen Weise umgesetzt.

Eine ebenfalls vorteilhafte Variante der Verfahrensweise a) besteht in der Umsetzung der Carbonsäuren der Formel VI mit 1 Mol Carbonyldiimidazol, wobei unter den angegebenen milden Reaktionsbedingungen in einem inerten polaren Lösungsmittel das aktivierte Carbonsäureimidazolid der Formel II mit X = 1-Imidazolyl gebildet wird, das unter analogen Bedingungen wie das oben beschriebene gemischte Säureanhydrid mit einem Amin der Formel III in die erfindungsgemäßen Verbindungen der Formel I übergeführt werden kann.

Eine weitere vorteilhafte Variante der Verfahrensweise a) besteht in der Umsetzung von Verbindungen der Formel II, worin $R_2$ bis $R_5$ und Y die angegebene Bedeutung besitzen und X eine niedere Alkoxygruppe mit 1 bis 6 C-Atomen im Alkylteil, bevorzugt Methoxy und Ethoxy, oder einen ggf. durch F, Cl oder Br substituierten Phenyloxyrest bedeutet, mit einem Amin der Formel III. Die Reaktion führt man vorteilhaft in Wasser oder einem polaren, gegenüber Aminen inerten organischen Lösungsmittel, beispielsweise in einem niederen Alkansäureamid, wie Dimethylformamid, Dimethylacetamid, Dimethylsulfoxid, in einem ringförmigen oder offenkettigen Ether oder Polyether, wie Tetrahydrofuran, Dioxan, Ethylenglycoldimethylether, bevorzugt aber in einem niederen Alkohol, wie Methanol, Ethanol, Propanol, Isopropanol oder in reinem unverdünntem Amin der Formel III ohne Anwendung eines Lösungsmittels aus. Man arbeitet bevorzugt in einem Temperaturbereich 10 bis 60 °C, besonders bevorzugt bei 15 bis 30 °C. Das Ende der Reaktion wird vorteilhaft dünnschichtchromatografisch an Kieselgel bestimmt. Die Reaktionsdauer beträgt erfahrungsgemäß je nach angewandter Reaktionstemperatur und Aminkomponente zwischen einer Stunde und 14 Tagen, beispielsweise bei Zimmertemperatur zwischen 5 und 72 Stunden. Die Carbonsäureester der Formel II und die Amine der Formel III werden bevorzugt in einem Molverhältnis von 1:1 bis 1:3 umgesetzt, wobei aber das Amin auch in einem bis zu 10fach molaren Überschuß angewendet werden kann. Die Verbindung der Formel II, worin $R_2$ bis $R_5$ und Y die angegebene Bedeutung besitzen und X für $-O-(C_1-C_6)$-Alkyl steht, gewinnt man in an sich bekannter Weise durch die Einwirkung eines niederen Alkanols mit 1 bis 6 C-Atomen bevorzugt von Methanol, Ethanol, Propanol, Isopropanol und Butanol, z.B. in Gegenwart eines organischen oder anorganischen Säurechlorides, durch Protonenkatalyse usw.

In gleicher Weise lassen sich ebenfalls die entsprechenden aktivierten Ester der Formel II mit X = CN, $N_3$, $-O-CH_2CN$ mit einem Amin der Formel III zur Reaktion bringen.

Die Amine der Formel III sind literaturbekannt.

Die Verbindungen der Formel VI mit M = H gewinnt man beispielsweise durch Friedel-Crafts-Reaktion einer aromatischen Verbindung VII mit Bernsteinsäureanhydrid zu Verbindungen VIII

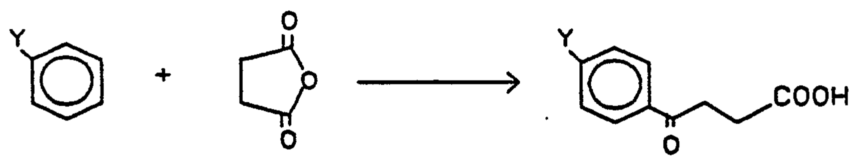

VII VIII

worin Y die angegebene Bedeutung besitzt, und durch anschließende Nitrierung, Reduktion, Diazotierung und Meerwein-Reaktion zur Verbindung IX

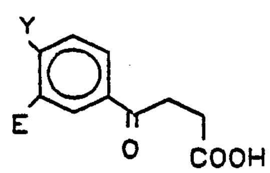

IX,

worin E eine $ClO_2S$-Gruppe ist und Y die angegebene Bedeutung besitzt.

Die Verbindungen der Formel IX werden nun in an sich bekannter Weise mit einem Aminphenol der Struktur V, worin $R_2$ bis $R_5$ die angegebene Bedeutung besitzen, unter Bildung der Verbindungen VI zur Reaktion gebracht. Dabei verfährt man bevorzugt so, daß man beide Reaktanten in einem Molverhältnis von 1:1 in Wasser oder einem inerten polaren organischen Lösungsmittel, wie sie oben unter Verfahrensvariante a) ausführlich aufgeführt werden, oder in Gemischen dieser Lösungsmittel mit Wasser zur Reaktion bringt und den Reaktionsablauf dünnschichtchromatografisch an Kieselgel als stationärer Phase verfolgt. Als vorteilhaft erweist sich die Anwesenheit von mindestens 1, besser von 2 oder mehr Molen, einer geeigneten protonenabfangenden Hilfsbase, wie sie bereits weiter oben ausführlich aufgezählt wurden, insbesondere von Pyridin oder Triethylamin, wobei die Reaktion zwischen -15 und +100 °C, vorteilhaft zwischen 20 und +80 °C durchgeführt wird.

Gemäß Verfahrensweise b) werden Sulfochloride der Formel IV mit den Aminophenolen der Formel V

zur Reaktion gebracht. Dabei verfährt man, wie bereits bei der Herstellung der Verbindungen IX beschrieben, so, daß man beide Reaktionspartner in einem Molverhältnis von 1:1 in Wasser oder einem inerten polaren organischen Lösungsmittel, wie Dimethylformamid, Dimethylacetamid, Dioxan, Tetrahydrofuran, Diethylenglycoldimethylether, in niederen Alkoholen mit 1-4 C-Atomen, z.B. in Methanol, Ethanol, Isopropanol, ebenso auch in einem Alkansäureniederalkylester, beispielsweise Essigsäuremethyl-oder -ethylester, in Gegenwart einer organischen oder anorganischen Hilfsbase zusammengibt. Dabei kommen die bereits oben erwähnten Basen in Betracht, wobei Trialkylamine wie beispielsweise Trimethylamin, Triethylamin oder Ethyldicyclohexylamin, N,N-Dimethylanilin, Pyridin, Alkali-und Erdalkalicarbonate oder -bicarbonate, wie Natriumcarbonat oder Natriumbicarbonat, oder Alkali-sowie Erdalkalisalze schwacher organischer Alkancarbonsäuren wie Natriumacetat bevorzugt verwendet werden. Die erwähnten Amine, beispielsweise Pyridin, können ebenfalls im Überschuß angewandt gleichzeitig als Lösungsmittel dienen. Die Reaktion kann in einem Temperaturbereich von -30 °C bis +100 °C, vorteilhaft zwischen +10 °C und +80 °C durchgeführt werden, wobei die Reaktionszeit im allgemeinen mindestens 30 Min. beträgt, längstens aber nach 2 Tagen beendet ist. Die Sulfonsäurehalogenide der Formel IV, worin Hal bevorzugt Chlor bedeutet, werden bevorzugt dadurch gewonnen, daß man eine 4-(3-Aminophenyl)-4-oxobuttersäure der Formel X beispielsweise wie oben beschrieben mit Hilfe der Methode der gemischten Anhydride mit Chlorameisensäureethylester in Gegenwart von Triethylamin aktiviert und mit einem Amin der Formel III in die 5-(3-Aminophenyl)-2-pyrrolidon-derivate der Formel XI überführt. Die Verbindungen der Formel XI werden anschließend bevorzugt in wäßriger Salzsäure und Natriumnitrit diazotiert und in situ sofort unter Verwendung von mit Schwefeldioxid gesättigter Eisessiglösung und Kupfer(II)chlorid im Sinne einer Meerwein-Reaktion in die Sulfochloride der Formel IV übergeführt.

**Reaktionsschema:**

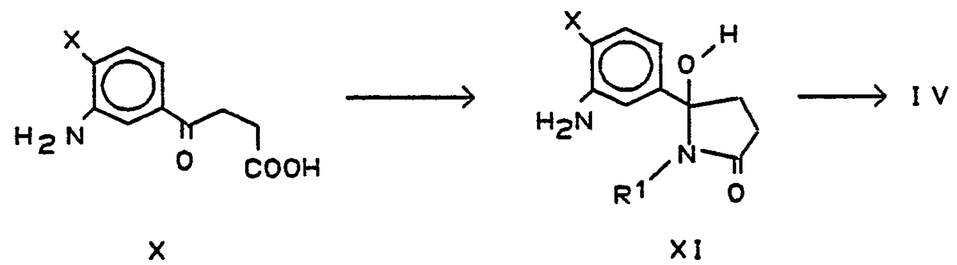

Erfindungsgemäß können außer den in den Ausführungsbeispielen beschriebenen Benzolsulfonamidderivaten beispielsweise auch die in der folgenden Tabelle zusammengestellten Verbindungen der allgemeinen Formel I bzw. Ia erhalten werden, wobei nachfolgend nur die Bezeichnung des Cyclotautometers der Formel I namentlich aufgeführt wird:

5-[4-Brom-3-(3,5-diisopropyl-4-hydroxyphenylsulfamoyl)phenyl]-5-hydroxy-1-methyl-2-pyrrolidon.
5-[4-Brom-3-(3,5-di-tert.butyl-4-hydroxyphenylsulfamoyl)phenyl]-5-hydroxy-1-methyl-2-pyrrolidon.
5-[3-(3,5-Diisopropyl-4-hydroxyphenylsulfamoyl)-4-trifluormethylphenyl]-5-hydroxy-1-methyl-2-pyrrolidon.
5-[3-(3,5-Di-tert.butyl-4-hydroxyphenylsulfamoyl)-4-trifluormethylphenyl]-5-hydroxy-1-methyl-2-pyrrolidon.
5-[4-Chlor-3-(3,5-di-tert.butyl-4-hydroxyphenylsulfamoyl)phenyl]-5-hydroxy-2-pyrrolidon.
5-[4-Chlor-3-(3,5-di-tert.butyl-4-hydroxyphenylsulfamoyl)phenyl]-1-ethyl-5-hydroxy-2-pyrrolidon.

Die Verfahrensprodukte sind wertvolle Arzneimittel und zeichnen sich durch eine Vielzahl sehr günstiger therapeutisch verwertbarer Eigenschaften, insbesondere durch die Kombination ihrer sehr guten diuretischen und saluretischen Wirkungen mit ausgeprägten antiatherosklerotischen Eigenschaften, aus.

Dies ist insbesondere deshalb von erheblichem therapeutischen Interesse, weil die bislang im Handel befindlichen Diusaluretika diejenigen Lipidanteile (VLDL-und LDL-Fraktionen) des Blutes, die für ein Auslösen oder Fortschreiten der Atherosklerose verantwortlich gemacht werden, entweder nicht vermindern oder sogar vermehren, so daß insbesondere im letzten Fall derartige Wirkstoffe ein gewisses atherogenes Potential besitzen.

Diuretika gehören auch noch heute wegen ihrer zahlreichen günstigen Eigenschaften und ihrer relativ guten Verträglichkeit zu den Basistherapeutika in der Bluthochdruckbehandlung. Da der Altersbluthochdruck zu einem wesentlichen Anteil Folge und Initiator atherosklerotischer Gefäßveränderung ist, die wiederum den Bluthochdruck verstärken, sind diusaluretische Wirkstoffe mit einer Verminderung des atherosklerotischen Risikos durch Senkung insbesondere der atherogenen LDL-Fraktion des Cholesterins im Blut von

besonderer therapeutischer Bedeutung.

Es war überraschend, daß die Verbindungen der Formel I, die sich von den bekannten Verbindungen der Formel XII durch Einführung eines substituierten Phenylrestes an der Sulfonamidgruppe unterscheiden, neben der diuretischen und saluretischen Wirkung eine den Serumspiegel der atherogenen Lipidfraktionen senkende Wirkung besitzen. Ein solches Wirkprofil bedeutet einen therapeutischen Fortschritt in der Behandlung der Hypertonie des älteren Menschen.

Aus zahlreichen Publikationen der letzten Jahre geht hervor, daß der Entstehung atherosklerotischer Plaques Verletzungen des Gefäßendothels vorausgehen. Derartige Endothelverletzungen können durch Fettsäureperoxide verursacht werden, so daß mit einer Verminderung dieser Lipidperoxide, die im wesentlichen in der LDL-Fraktion transportiert werden, zwangsläufig eine Verminderung des atherogenen Risikos verbunden ist. Es konnte nun gezeigt werden, daß die erfindungsgemäßen Verbindungen die Bildung von Malondialdehyd, der ein Maß für die Lipidperoxidation ist, zu unterdrücken vermögen ($IC_{50}$ = 5 x $10^{-6}$ Mol/l). Diese antioxidative Wirkkomponente läßt sich auf den Hydroxyphenylamino-Substituenten an der Sulfonylgruppe der Verbindungen I zurückführen.

Überraschend stark ausgeprägt zeigt sich die antihypertensive Wirkung der erfindungsgemäßen Verbindungen, die sich in vitro auf den Gefäßmuskel (isolierter Aortenstreifen) nachweisen läßt. Die Verbindungen hemmten am isolierten Aortenstreifen die noradrenalin-induzierten Kontraktionen mit einer $IC_{50}$ = $10^{-4}$ Mol/l. An diesem Modell gehören sie zu den am stärksten gefäßrelaxierenden Diuretika.

Somit vermindern die erfindungsgemäßen Verbindungen nicht nur das Fortschreiten der Atherosklerose durch Reduktion der atherogenen Cholesterinfraktion und durch die Verminderung des erhöhten Blutdrucks, sondern wirken darüberhinaus durch die Zerstörung der Lipidperoxide präventiv einer Neubildung atherosklerotischer Plaques entgegen.

Die neuen Verfahrensprodukte zeigen an der Ratte und an Affen eine mit handelsübliche Diuretika wie Hydrochlorothiazid und Chlorthalidon vergleichbare salidiuretische Wirkung. Darüberhinaus zeichnen sich die die neuen Verfahrenserzeugnisse durch eine langanhaltende Wirkungsdauer aus. Deshalb sind die neuen Verfahrensprodukte auch infolge ihrer diusaluretischen Wirkung zur Behandlung hypertoner Zustände geeignet, wobei man sie, wie heute allgemein üblich, gegebenenfalls mit einem anderen Antihypertonikum kombinieren wird.

Die Verbindungen I werden in Dosen von mindestens 0,5 mg/kg und Tag, bevorzugt 1 mg/kg und Tag bis höchstens 10 mg/kg/Tag, vorzugsweise bis 5 mg/kg/Tag angewendet, bezogen auf einen erwachsenen Menschen von etwa 75 kg Körpergewicht.

Als therapeutische Zubereitung der neuen Verbindungen kommen vor allem Tabletten, Dragees, Kapseln und Suppositorien sowie auch Ampullen zur parenteralen Verabreichtung (i.v., s.c., und i.m.) infrage. Die therapeutische Einheitsdosis liegt zwischen 0,5 und 500 mg, vorzugsweise zwischen 10 und 300 mg pro Tablette.

Diese Zubereitungen können speziell bei der Behandlung des Bluthochdrucks außer den üblichen Füll- und Trägerstoffen noch ein Antihypertensivum, wie beispielsweise Reserpin, Hydralazin, Guanethidin, α-Methyldopa, Clonidin, einen β-sympathikolytischen Wirkstoff, wie beispielsweise Propanolol, oder einen ACE-Inhibitor, wie Captopril, Enalapril oder Ramipril enthalten.

Außerdem sind therapeutische Kombinationspräparate mit kaliumretinierenden Verbindungen, wie Aldosteronantagonisten, z.B. Spironolacton oder Pseudoaldosteronantagonisten wie Triamteren oder Amilorid von Interesse. Weiterhin kommt Kalium-Substitution in verschiedenen Anwendungsformen, z.B. Dragees, Tabletten, Brausetabletten, Säften u.a. infrage.

Von therapeutischem Interesse können ebenfalls Kombinationen der erfindungsgemäßen Verbindungen mit einem antihyperurikämisch und/oder urikosurisch wirksamen Mittel sein, das entweder durch eine Hemmung der Xanthinoxidase oder durch Erhöhung der renalen Harnsäureausscheidung stärkere Blutharnsäureerhöhungen vermeidet.

In den nachfolgenden Beispielen sind die Schmelz-und Zerstezungspunkte der Ausführungsbeispiele nicht korrigiert.

**Beispiel 1**

5-[4-Chlor-3-(3,5-diisopropyl-4-hydroxyphenylsulfamoyl)phenyl]-5-hydroxy-1-methyl-2-pyrrolidon.

a) 5-(3-Amino-4-chlorphenyl)-5-hydroxy-1-methyl-2-pyrrolidon

Zu einer Lösung von 60 g 4-(3-Amino-4-chlorphenyl)-4-oxobuttersäure und 27,8 g Triethylamin in 500 ml Tetrahydrofuran dosiert man 42,8 g Chlorameisensäureethylester so zu, daß die Temperatur 10 °C nicht überschreitet. Man rührt weitere 10 Min. im Eisbad und versetzt sodann unter Beibehaltung der Kühlung mit einer Lösung aus 35 g gasförmigem Methylamin in 250 ml Tetrahydrofuran, wobei eine Temperatur von 25 °C nicht überschritten werden soll. Nach weiterem Rühren über 45 Minuten im Eisbad destilliert man das Lösungsmittel ab, versetzt den Rückstand mit Wasser und filtriert den kristallinen Niederschalg ab. Kristalle, Fp. 154 °C (aus Ethanol).

b) 5-(4-Chlor-3-chlorsulfonylphenyl)-5-hydroxy-1-methyl-2-pyrrolidon

Zu einer Lösung von 30, 5 g 5-(3-Amino-4-chlorphenyl)-5-hydroxy-1-methyl-2-pyrrolidon in 300 ml halbkonzentrierte Salzsäure dosiert man bei 0 bis 5 °C eine Lösung von 9,9 g Natriumnitrit in 60 ml Wasser unter die Oberfläche. Man rührt weitere 10 Min. unter Kühlung nach und versetzt das Reaktionsgemisch sodann portionsweise mit einer Mischung aus 15,2 g Kupfer(II)chlorid Dihydrat und 450 ml einer mit Schwefeldioxid gesättigten Eisessiglösung. Man rührt weitere 30 Min., fällt das Sulfochlorid sodann durch Zugabe von Wasser aus und filtriert die Kristalle ab. Fp. 137-139 °C.

c) 5-(4-Chlor-3-(3,5-diisopropyl-4-hydroxyphenylsulfamoyl)phenyl)-5-hydroxy-1-methyl-2-pyrrolidon

Zu einer Lösung von 17,5 g 5-(4-Chlor-3-chlorsulfonylphenyl)5-hydroxy-1-methyl-2-pyrrolidon und 22,7 g Triethylamin in 200 ml Essigsäureethylester fügt man portionsweise eine Suspension von 29,1 g 4-Amino-2,6-diisopropylphenol in 450 ml Essigester. Man rührt 3 Stunden bei 50 °C, kühlt ab, versetzt mit Wasser, trennt die organische Phase ab und extrahiert die wäßrige Phase mehrmals mit Essigester. Die vereinigten organischen Phasen werden mit Wasser gewaschen, über Natriumsulfat getrocknet, und das Lösungsmittel wird abdestilliert. Der amorphe rote Rückstand wird nach Zugabe von etwa 250 ml Methylenchlorid zum Rückfluß erhitzt und durch Anreiben zur Kristallisation gebracht. Man rührt 30 Min. bei Zimmertemperatur, filtriert den Niederschlag ab und kristallisiert ohne längeres Stehenlassen aus Acetonitril um. Farblose Kristalle, Fp. 198-200 °C.

**Beispiel 2**

5-[4-Chlor-3-(3,5-Di-tert.butyl-4-hydroxyphenylsulfamoyl)phenyl]-5-hydroxy-1-methyl-2-pyrrolidon.

Erhält man analog der in Beispiel 1c) angegebenen Vorschrift durch Umsetzung von 5-(4-Chlor-3-chlorsulfonylphenyl)-5-hydroxy-1-methyl-2-pyrrolidon mit 4-Amino-2,6-di-tert.butylphenol in Gegenwart von Triethylamin. Farbloser Feststoff, Fp. 120-130 °C.

**Beispiel 3**

5-[4-Chlor-3-(3-tert.butyl-4-hydroxy-5-methylphenylsulfamoyl)phenyl]-5-hydroxy-1-methyl-2-pyrrolidon

erhält man analog der in Beispiel 1c) angegebenen Vorschrift durch Umsetzung von 5-(4-Chlor-3-chlorsulfonylphenyl)-5-hydroxy-1-methyl-2-pyrrolidon mit 4-Amino-2-tert.butyl-6-methylphenol in Gegenwart von Triethylamin. Farbloser Feststoff, Fp. 120 °C.

## Ansprüche

1. Benzolsulfonamidderivate der Formel I

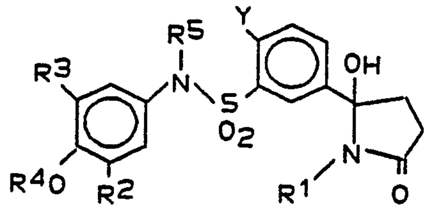

I,

worin
$R^1$ Wasserstoff, Alkyl oder Alkenyl mit 1-4 C-Atomen, Cycloalkyl mit 3 bis 5 Ringgliedern,
$R^2$ Wasserstoff oder einen Alkylrest mit 1 bis 6 C-Atomen,
$R^3$ einen Alkylrest mit 3 bis 5 C-Atomen,
$R^4$ und $R^5$ Wasserstoff, einen ($C_1$-$C_3$)-Alkylrest oder einen Acylrest einer aliphatischen Carbonsäure mit 1 bis 3 C-Atomen und
Y Wasserstoff, Methyl, Trifluoromethyl, F oder Cl bedeuten, sowie die entsprechend zu I offenkettigen tautomeren Formen der Formel Ia

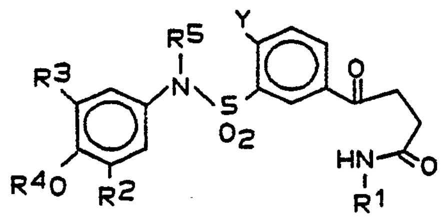

Ia

worin $R^1$ bis $R^5$ und Y die angegebene Bedeutung besitzen.

2. Verbindungen I nach Anspruch 1, dadurch gekennzeichnet, daß mindestens einer der Substituenten folgende Bedeutung hat:
$R^1$ Wasserstoff, Alkyl oder Alkenyl mit 1-4 C-Atomen, Cycloalkyl mit 3 bis 5 Ringgliedern,
$R^2$ Wasserstoff oder ein Alkylrest mit 1 bis 6 C-Atomen,
$R^3$ ein Alkylrest mit 3 bis 5 C-Atomen,
$R^4$ Wasserstoff,
$R^5$ Wasserstoff,
Y Chlor.

3. Verbindungen I nach Anspruch I, dadurch gekennzeichnet, daß mindestens einer der Substituenten folgende Bedeutung hat:
$R^1$ Wasserstoff, Alkyl oder Alkenyl mit 1-4 C-Atomen, Cycloalkyl mit 3 bis 5 Ringgliedern,
$R^2$ Methyl, Isopropyl, t-Butyl,
$R^3$ Isopropyl, t-Butyl,
$R^4$ Wasserstoff,
$R^5$ Wasserstoff,
Y Chlor.

4. Verbindungen I nach Anspruch 1, dadurch gekennzeichnet, daß mindestens einer der Substituenten folgende Bedeutung hat:
$R^1$ Methyl
$R^2$ Methyl, Isopropyl, t-Butyl,
$R^3$ Isopropyl, t-Butyl,
$R^4$ Wasserstoff,
$R^5$ Wasserstoff,
Y Chlor.

5. Verbindung I nach Anspruch 1, dadurch gekennzeichnet, daß sie aus der Gruppe
5-[4-Chlor-3-(3,5-diisopropyl-4-hydroxyphenylsulfamoyl)phenyl]-5-hydroxy-1-methyl-2-pyrrolidon und
5-[4-Chlor-3-(3,5-di-tert.-butyl-4-hydroxyphenylsulfamoyl)phenyl]-5-hydroxy-1-methyl-2-pyrrolidon ausgewählt ist.

6. Verfahren zum Herstellen der Verbindungen I nach Anspruch 1, dadurch gekennzeichnet, daß man
a) Verbindungen der Formel II

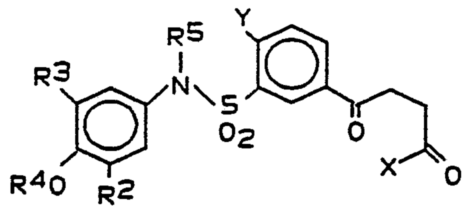

II

mit einem primären Amin der Formel III un üblicher Weise umsetzt

$R^1$-$NH_2$ III

wobei $R^1$ bis $R^5$ und Y die angegebene Bedeutung besitzen und X der reaktive anorganische oder organische Rest der Carbonsäure (X = OH) ist, oder
b) Verbindungen der Formel IV

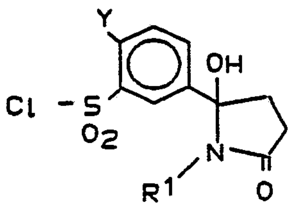

IV

mit einem Amin der Formel V

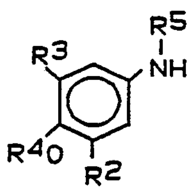

V

umsetzt, worin $R^1$ bis $R^5$ und Y die angegebene Bedeutung besitzen,
und gegebenenfalls die nach Weg a) und b) erhaltenen Verbindungen der Formel I, worin $R^4$ und/oder $R^5$ Wasserstoff bedeutet, durch übliche Alkylierung oder Acylierung in Verbindungen der Formel I überführt, in denen $R^4$ und/oder $R^5$ eine der weiteren oben angegebenen Bedeutungen hat.

7. Verwendung einer Verbindung I nach Anspruch 1 als Diuretikum und Antihypertensivum mit lipidsenkender Wirkung.

8. Verwendung einer Verbindung I nach Anspruch 1 zur Herstellung eines Medikaments mit diuretischer, antihypertensiver und lipidsenkender Wirkung.

9. Pharmazeutische Zubereitung bestehend aus einer wirksamen Menge einer Verbindung I nach Anspruch 1 und pharmazeutisch üblichen Zusatzstoffen.

Patentansprüche für die folgenden Vertragsstaaten: ES und GR

1. Verfahren zum Herstellen von Benzolsulfonamidderivaten der Formel I

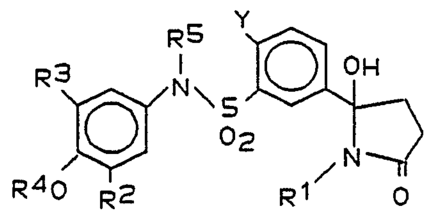

worin
$R^1$ Wasserstoff, Alkyl oder Alkenyl mit 1-4 C-Atomen, Cycloalkyl mit 3 bis 5 Ringgliedern,
$R^2$ Wasserstoff oder einen Alkylrest mit 1 bis 6 C-Atomen,
$R^3$ einen Alkylrest mit 3 bis 5 C-Atomen,
$R^4$ und $R^5$ Wasserstoff, einen $(C_1-C_3)$-Alkylrest oder einen Acylrest einer aliphatischen Carbonsäure mit 1 bis 3 C-Atomen und
Y Wasserstoff, Methyl, Trifluormethyl, F oder Cl bedeuten, sowie der entsprechend zu I offenkettigen tautomeren Formen der Formel Ia

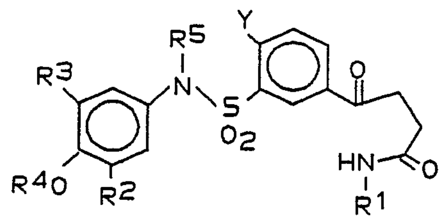

Ia

worin $R^1$ bis $R^5$ und Y die angegebene Bedeutung besitzen. dadurch gekennzeichnet, daß man
a) Verbindungen der Formel II

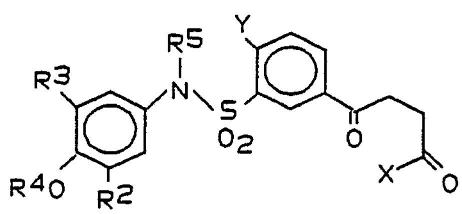

II

mit einem primären Amin der Formel III in üblicher Weise umsetzt
$R^1$-$NH_2$ III
wobei $R^1$ bis $R^5$ und Y die angegebene Bedeutung besitzen und X der reaktive anorganische oder organische Rest der Carbonsäure (X = OH) ist, oder
b) Verbindungen der Formel IV

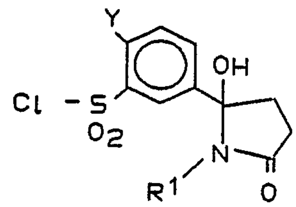

IV

mit einem Amin der Formel V

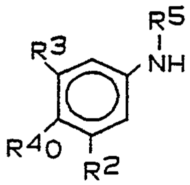

V

umsetzt, worin $R^1$ bis $R^5$ und Y die angegebene Bedeutung besitzen, und gegebenenfalls die nach Weg a) und b) erhaltenen Verbindungen der Formel I, worin $R^4$ und/oder $R^5$ Wasserstoff bedeutet, durch übliche Alkylierung oder Acylierung in Verbindungen der Formel I überführt, in denen $R^4$ und/oder $R^5$ eine der weiteren oben angegebenen Bedeutungen hat.

2. Verfahren nach Anspruch 1, dadurch gekennzeichnet, daß mindestens einer der Substituenten folgende Bedeutung hat:

$R^1$ Wasserstoff, Alkyl oder Alkenyl mit 1-4 C-Atomen, Cycloalkyl mit 3 bis 5 Ringgliedern,
$R^2$ Wasserstoff oder ein Alkylrest mit 1 bis 6 C-Atomen,
$R^3$ ein Alkylrest mit 3 bis 5 C-Atomen,
$R^4$ Wasserstoff,
$R^5$ Wasserstoff,
Y Chlor.

3. Verfahren nach Anspruch 1, dadurch gekennzeichnet, daß mindestens einer der Substituenten folgende Bedeutung hat:

$R^1$ Wasserstoff, Alkyl oder Alkenyl mit 1-4 C-Atomen, Cycloalkyl mit 3 bis 5 Ringgliedern,
$R^2$ Methyl, Isopropyl, t-Butyl,
$R^3$ Isopropyl, t-Butyl,
$R^4$ Wasserstoff,
$R^5$ Wasserstoff,
Y Chlor.

4. Verfahren nach Anspruch 1, dadurch gekennzeichnet, daß mindestens einer der Substituenten folgende Bedeutung hat:

$R^1$ Methyl
$R^2$ Methyl, Isopropyl, t-Butyl,
$R^3$ Isopropyl, t-Butyl,
$R^4$ Wasserstoff,
$R^5$ Wasserstoff,
Y Chlor.

5. Verfahren nach Anspruch 1, dadurch gekennzeichnet, daß eine Verbindung aus der Gruppe
5-[4-Chlor-3-(3,5-diisopropyl-4-hydroxyphenylsulfamoyl)phenyl]-5-hydroxy-1-methyl-2-pyrrolidon und
5-[4-Chlor-3-(3,5-di-tert.-butyl-4-hydroxyphenylsulfamoyl)phenyl]-5-hydroxy-1-methyl-2-pyrrolidon hergestellt wird.

6. Verwendung einer Verbindung I nach Anspruch 1 als Diuretikum und Antihypertensivum mit lipidsenkender Wirkung.

7. Verwendung einer Verbindung I nach Anspruch 1 zur Herstellung eines Medikaments mit diuretischer, antihypertensiver und lipidsenkender Wirkung.